# EUROPEAN PATENT APPLICATION

(11) **EP 3 786 971 A1**
(43) Date of publication of application: **03.03.2021**
(21) Application number: 20194130.9
(22) Date of filing: 02.09.2020
(51) Int. Cl.: G16H 20/30, A63B 24/00, G16H 50/20

(54) **ADVANCEMENT MANAGER IN A HANDHELD USER DEVICE**

(30) Priority: 02.09.2019 SE 1950996
(71) Applicant: Always Exploring AB, 111 37 Stockholm (SE)
(72) Inventor: KARLSSON, Martin, 111 37 Stockholm (SE)
(74) Representative: Kransell & Wennborg KB

(57) **Abstract**

A method for an advancement manager in a handheld user device is presented. The method is performed in a handheld user device (1). The method comprises detecting (S100) an exercise of a person in front of a camera (46) of the handheld user device, wherein the exercise is a trained exercise, determining (S130), when a stability of the detected exercise is within a first threshold, a performance of the detected exercise as correct, and otherwise determine the performance as incorrect, verifying (S140) that the performance determined as incorrect occurs in a subsequent repetition of the detected exercise before providing the correction indication, and providing (S150) a correction indication towards a user interface, UI, of the handheld user device for the determined incorrect performance. A user device and a computer program for an advancement manager in a handheld user device are also presented.

## Description

### TECHNICAL FIELD

The present disclosure presents a method, a handheld user device, and a computer program for an advancement manager in a handheld user device.

### BACKGROUND

Many people use personal trainers to get feedback on how they train or should train. With artificial intelligence it is possible to create a virtual personal trainer to get feedback on training. Similar situations may e.g. arise also for physiotherapy.

One problem is however how to virtually determine physiologically correct training.

### SUMMARY

One objective is to enable physiologically correct training of an exercise with the aid of a virtual trainer.

According to a first aspect there is presented a method for an advancement manager in a handheld user device. The method is performed in a handheld user device. The method comprises detecting an exercise of a person in front of a camera of the handheld user device, wherein the exercise is a trained exercise, determining, when a stability of the detected exercise is within a first threshold, a performance of the detected exercise as correct, and otherwise determine the performance as incorrect, verifying that the performance determined as incorrect occurs in a subsequent repetition of the detected exercise before providing a correction indication, and providing the correction indication towards a user interface (UI) of the handheld user device for the verified incorrect performance.

By providing a correction indication to a person doing an exercise, when the exercise is not performed sufficiently stable, a physiologically correct training is achievable.

The method may further comprise determining a weight of an exercise equipment present in the detected exercise, wherein the first threshold is dependent of the determined weight.

The exercise may be detected by image recognition.

The stability may be determined by detecting movement of one or more reference points of the person in front of the camera. The first threshold may be a distance unit in a first dimension based on a normalized value of a distance in a second dimension.

The performance of the detected exercise may be determined as correct when also a speed of the detected exercise is below a second threshold. The second threshold may be a predetermined value.

A performance of the detected exercise may be determined as correct when also a relative position of two or more reference points of the person in front of the camera is within a predetermined interval.

The first threshold may dependent of an exercise history of the person in front of the camera.

The correction indication may be an image, or a video, displayed on a display of the handheld user device, and/or a voice message played by a speaker of the handheld user device.

The method may further comprise providing an advancement indication towards the UI for the detected exercise.

A set of repetitions of the detected exercise may be started upon the detected exercise.

According to a second aspect there is provide a handheld user device for an advancement manager. The handheld user device comprises a camera, a UI, a processing circuitry, and a computer program product. The computer program product stores instructions that, when executed by the processing circuitry, causes the handheld user device to detect an exercise of a person in front of the camera, wherein the exercise is a trained exercise, determine, when a stability of the detected exercise is within a first threshold, a performance of the detected exercise as correct, verify that the performance determined as incorrect occurs in a subsequent repetition of the detected exercise before providing a correction indication, and otherwise determine the performance as incorrect, and to provide the correction indication towards the UI for the verified incorrect performance.

According to a third aspect there is provided a computer program for an advancement manager in a handheld user device. The computer program comprises computer program code which, when run in a handheld user device, causes the handheld user device to detect an exercise of a person in front of a camera of the handheld user device, wherein the exercise is a trained exercise, determine, when a stability of the detected exercise is within a first threshold, a performance of the detected exercise as correct, verifying that the performance determined as incorrect occurs in a subsequent repetition of the detected exercise before providing a correction indication, and otherwise determine the performance as incorrect, and to provide a correction indication towards a user interface, UI, of the handheld user device for the verified incorrect performance.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated.

### BRIEF DESCRIPTION OF THE DRAWINGS

Aspects and embodiments are now described, by way of example, with reference to the accompanying drawings, in which:
Fig. 1 shows a diagram schematically illustrating a setup wherein embodiments presented herein can be applied;
Fig. 2 is a flowchart schematically illustrating embodiments of methods presented herein;
Fig. 3 is a diagram schematically illustrating a handheld user device with a display of an advancement manager according to an embodiment presented herein;
Fig. 4 is a diagram schematically illustrating communication between parts of a handheld device according to an embodiment presented herein;
Figs. 5 and 6 are diagrams schematically illustrating some components of devices presented herein; and
Figs. 7 and 8 are diagrams schematically illustrating functional modules of devices presented herein.

### DETAILED DESCRIPTION

The aspects of the present disclosure will now be described more fully hereinafter with reference to the accompanying drawings, in which certain embodiments of the invention are shown.

These aspects may, however, be embodied in many different forms and should not be construed as limiting; rather, these embodiments are provided by way of example so that this disclosure will be thorough and complete, and to fully convey the scope of all aspects of invention to those skilled in the art. Like numbers refer to like elements throughout the description.

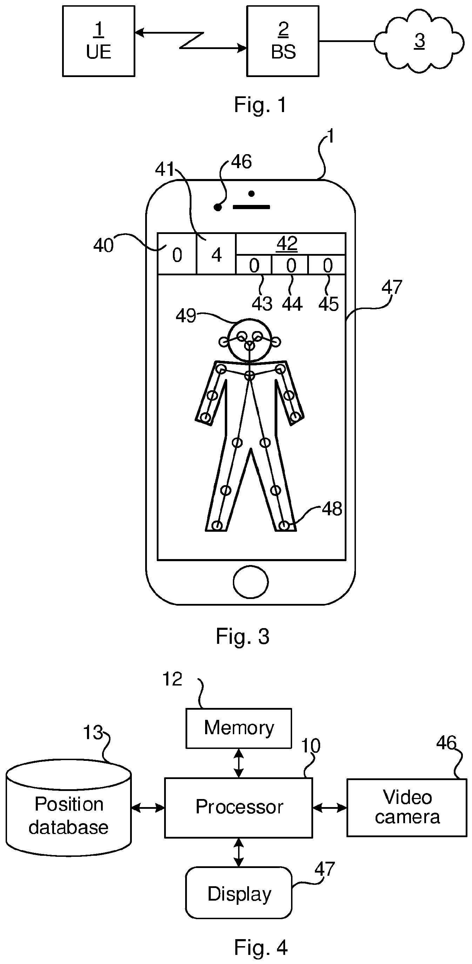

FIG. 1 shows an environment wherein methods presented herein may be implemented. A handheld user device 1 is in connectivity with a base station 2 of a radio communication network, which in turn is connected to the Internet 3, which e.g. allows use of cloud services for the handheld user device 1. The base station 2 may e.g. be part of a cellular network such as 4G, or part of a wireless connection such as WiFi.

A virtual trainer is implemented by an interactive program run on the handheld user device 1. The handheld user device is a wireless device, such as a smartphone. The interactive program may e.g. be controlled by voice, gestures, or touch controls, or a combination thereof. A workout program, including a collection of exercises, may be selected by a user. An exercise may e.g. be split squats, shoulder press or kettlebell swings. Alternatively, the user may start working out by doing one or more exercises of a workout program, which are identified by the virtual trainer by a combination of pose estimation and object tracking.

The user positions the handheld user device 1 on a wall or other surface, or on a tripod, such that a camera of the handheld user device 1 can record a workout program of the user in front of the camera. A sticky glass or mirror case, such as a nanocase may be used to position the handheld user device 1 on a wall or other surface. The user then positions himself in front of the camera and starts the virtual trainer, to e.g. train a set of split squats. The virtual trainer may be configured to instruct the user to stand in front of the camera, such that relevant reference points of the user can be identified during exercises. Such reference points may include nose, neck, right shoulder, right elbow, right hand, left shoulder, left elbow, left hand, right hip, right knee, right foot, left hip, left knee, left foot, right eye, left eye, right ear and left ear. A positioning system may for example aim to position the user such that all or a certain subset of the previous joints can be identified.

An exercise may comprise one or more of a set of a specific number of repetitions, e.g. 10 repetitions of lunges. An exercise may alternatively comprise one or more of a set of as many repetitions as possible during a specific time period, e.g. as many squat jumps as possible during 60 s. In yet an alternative, an exerciser may comprise a specific number of repetitions during a specific time period, wherein any time left after the specific number of repetitions have been performed can be used to rest, e.g. perform 4 rounds of 10 push-ups within a minute and rest for the remainder of each minute.

A specific time period of rest may be added after each set, before a workout is advanced to a subsequent set. The subsequent set may be started when a first repetition of that expected exercise is detected. The user may be prompted to start the expected exercise after a time period of rest or the preceding exercise was completed.

Advancement indications may be provided to the user whether the performance is determined correct or not.

The camera may be a video camera, such as an RGB video camera. The handheld user device 1 may be connected to a server in the cloud, the server including a database of workout program movements or exercises that is used to detect and perform workout program or exercise recognition. The movement database stores sequences of images and threshold values for particular series of workout programs or exercises used by the virtual trainer. The workout program or exercise movement database can be shared by other interactive training servers to improve machine learning identification.

The database contains descriptions on when to determine movements of a workout program or an exercise to be physiologically correct. These descriptions can be either in the form of rules where one or more reference point positions are expected to be positioned in relation to another position, or in the form of machine learned models that has been trained to identify when an advancement or repetition should be deemed made.

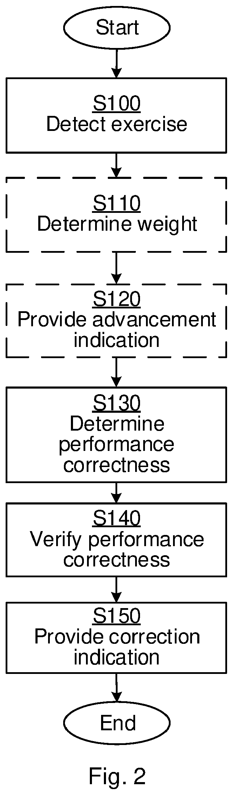

According to an aspect a method for an advancement manager in a handheld user device is presented with reference to Fig. 2. The method is performed in a handheld user device 1. In processing block S100 an exercise of a person in front of a camera of the handheld user device is detected. The exercise is a trained exercise. In optional processing block S110 a weight of an exercise equipment present in the detected exercise is determined. The first threshold is dependent of the determined weight. In optional processing block S120 an advancement indication is provided towards a user interface (UI) of the handheld user device for the detected exercise. In processing block S130, when a stability of the detected exercise is within a first threshold, a performance of the detected exercise is determined as correct, and otherwise the performance is determined as incorrect. In processing block S140 it is verified that the performance determined as incorrect in processing block S130 occurs also in a subsequent repetition of the detected exercise before providing a correction indication in processing block S150. In processing block S150 the correction indication is provided towards the UI of the handheld user device for the verified incorrect performance.

The exercise may e.g. be detected by image recognition and also by pose position identification, object identification and tracking, a combination thereof, or by manual entry into the virtual trainer. Image recognition may be used to joints in a picture, and a sequence of such pictures are analysed to recognise an exercise.

The stability may in processing block S130 be determined by detecting movement of one or more reference points of the person in front of the camera in two dimensions x and y. The detection of movement may be made by image recognition. The first threshold may e.g. be a distance unit in the x dimension based on a normalized value of a distance in the y dimension, or vice versa. The normalized value in the y dimension may be a normalized distance between two or more joints. For a shoulderpress exercise, where the wrist moves less than e.g. 0.2 of the elbow to wrist distance in the x dimension, the exercise may be determined to be stable and therefore correctly performed.

The performance of the detected exercise may in processing block S130 be determined as correct when also a speed of the detected exercise is below a second threshold. The speed may be determined by detecting the position of a reference point of the person in front of the camera and detecting the number of pixels moved per second in the x or y dimension or Euclidean distance. The detection of number of moved pixels may be made by image recognition. The second threshold may be a predetermined value based on training knowledge.

A performance of the detected exercise may in processing block S130 be determined as correct when also a relative position of two or more reference points of the person in front of the camera is within a predetermined interval. The predetermined interval may be based on training knowledge.

The first threshold may be dependent of an exercise history of the person in front of the camera.

The correction indication may in processing block S150 be an image, or a video, displayed on a display of the handheld user device, and/or a voice message played by a speaker of the handheld user device.

When an exercise has been detected, a repetition number may be increased in processing block S120. When a predetermined number of repetitions have been reached, a first set is completed. A second set of repetitions may follow. The predetermined number of repetitions may initially be selected for each exercise, and later be adjusted by the advancement manager. Detection of an exercise may e.g. be by detecting a movement of a reference point such as the nose. The movement may be a distance e.g. from y to y-0.2.

A specific time period of rest may be added after each set, before a workout is advanced to a subsequent set. The subsequent set may be started when a first repetition of an expected exercise is detected in processing block S110. The user may be prompted to start the expected exercise after a time period of rest or the preceding exercise was completed.

Advancement indications may be provided to the user whether the performance is determined correct or not.

When an exercise is determined as incorrect, due to that the stability being over the first threshold, a corrective information may be sent to the UI to be visually shown on the display and/or via voice, e.g. stating "Please keep your feet hip width apart". When an exercise is determined as incorrect, due to that the speed is above the second threshold, a corrective information may be visually shown on the display and/or via voice, e.g. illustrating a speed meter or stating "Please slow down pace of repetitions". When an exercise is determined as incorrect, due to that a relative position of two or more reference points of the person in front of the camera is outside a predetermined interval, a corrective information may be visually shown on the display and/or via voice, e.g. stating "Keep your knee above your foot".

A user may choose to display a camera view at any time which shows the image the camera is capturing. In addition, the user may opt to show the pose skeleton imposed on top of the body.

The operations shown in Fig. 2 will now be illustrated and described in more detail in conjunction with Figs. 3-4.

Fig. 3 illustrates a smartphone 1 with a camera 46 and a display 47. The display 47 illustrates a picture of how the advancement manager may be displayed. A first box 40 shows the number of correctly performed movements of the exercise, e.g. split squats. A second box 41 shows the weight of training equipment when used. The weight may e.g. be identified via image recognition or via manual entry. The third box 43 shows which exercise that is being performed. A fourth 44, a fifth 45 and a sixth 46 box show how many repetitions have been correctly performed for corresponding first, second and third training set of the exercise. The rest of the display shows an image of the person 49 standing in front of the camera 46. An overlay over the image of the person 49 shows points 48 of the person identified by the virtual trainer. The points include nose, neck, right shoulder, right elbow, right hand, left shoulder, left elbow, left hand, right hip, right knee, right foot, left hip, left knee, left foot, right eye, left eye, right ear and left ear of the person 49.

Fig. 4 illustrates some functional blocks of the virtual trainer. A processor and/or a graphical processing unit 10 of the smartphone 1 is connected to a memory 12, which is used to record video of the video camera 46 to be displayed on the display 47. The processor is also connected to an exercise database 13, which is used to determine exercise advancement and to store exercise history.

The exercise database can be used to classify a user's movements between pose positions as a particular exercise, by comparing the user's movements to a known set of exercise catalogued and stored in an exercise library. For each exercise in the library, baseline or "ground truth" data has first been generated in a preprocessing step for each exercise. The "ground truth" data can then be used as a baseline against which a user's movements are compared in order to classify the movements as a particular exercise. Data characterizing the relative positions of the feature points of interest over several images in a sequence can be used for the comparison. Each exercise in the library may have a sequence of images covering a period of time required to perform the exercise, with a uniform time lapse occurring between images in the sequence. Each exercise may be associated with a minimum number of sequential images sufficient to capture the entire movement of the exercise. Thus, a quick movement like a finger snap requires fewer sequential images, while a movement that takes a longer time to perform, for example, a handshake, requires more sequential images. The exercise should e.g. differentiate for non-exercise activities such as drinking water or walking around. This may e.g. be performed by detecting an exercise' start position and end position, and when a user is not in motion therebetween any movement is ignored.

When an exercise is determined to be within the first threshold optionally below the second threshold, and optionally within the interval, an advancement may be determined to have been made. However, when an exercise is matched to a trained exercise with a difference over one or more thresholds, the exercise is determined to not have been correctly performed, i.e. being incorrect. This may result in that an advancement of the exercise will not be displayed or may result in that a counter for the exercise is advanced similarly as for a correct exercise. An overlay may be presented to indicate the incorrectness by colour or other highlighting. The display of the recorded person and the overly may alternatively only display the overlay when incorrectly performed.

An exercise may be considered as movements between two different exercise positions. An exercise may be considered incorrect when one of the two exercise positions are incorrect. The feet should e.g. be in line with the shoulders and hips, before a squat exercise. An exercise may also be considered incorrect when one reference point is incorrect with regards to another reference point. The knee should e.g. be outside the vertical ankle hip line during a squat exercise.

Exercise details, such as number of repetitions and equipment weight, may be selected or may be recommended the virtual trainer, e.g. based on weight, length, age of the person in front of the camera and more importantly on historic data such as weights, number of repetitions, speed and stability for previous exercises. For example, if a user has managed to do 10 squats with a 20kg kettlebell 3 workouts in a row, it may be time to increase to 12 reps or increase to 22 kg. This information may be stored in a database for each user after each workout. A progression manager program may calculate the appropriate weight and number of repetitions before each exercise.

An exercise recommendation for a person may be based or adjusted on observed movability, weaknesses and/or imbalances.

An exercise may comprise one or more of a set of a specific number of repetitions, e.g. 10 repetitions of lunges. An exercise may alternatively comprise one or more of a set of as many repetitions as possible during a specific time period, e.g. as many squat jumps as possible during 60 s. In yet an alternative, an exerciser may comprise a specific number of repetitions during a specific time period, wherein any time left after the specific number of repetitions have been performed can be used to rest, e.g. perform 4 rounds of 10 push-ups within a minute and rest for the remainder of each minute.

A specific time period of rest may be added after each set, before a workout is advanced to a subsequent set. The subsequent set may be started when a first repetition of an expected exercise is detected.

Advancement indications may be provided to the user whether the performance is determined correct or not.

According to an aspect an embodiment of a handheld user device for an advancement manager is presented with reference to Fig. 4. The handheld user device 1 comprises a camera 46, a UI 11, a processing circuitry 10, and a computer program product. The computer program product stores instructions that, when executed by the processing circuitry, causes the handheld user device to detect an exercise of a person in front of the camera, wherein the exercise is a trained exercise, determine, when a stability of the detected exercise is within a first threshold, a performance of the detected exercise as correct, and otherwise determine the performance as incorrect, verifying that the performance determined as incorrect occurs in a subsequent repetition of the detected exercise before providing a correction indication, and to provide the correction indication towards the UI for the verified incorrect performance.

The handheld user device may be used with or without cordless headphones.

Exercise history may be obtained by accessing a database comprising data such as each users weight and number of repetitions performed for each exercise performed previously.

The stability may be determined by analysing relevant joints movement smoothness. For example, in a shoulder press, if one arm is not able to push up the weight at the same speed as the other or that the path the weight takes is different from the first repetitions, the exercise may be determined not sufficiently stable.

The speed may be determined by measuring the movement of a joint in x/y in number of pixels per time unit.

When an exercise advancement is determined correct, a repetition number may be increased on the display. When a selected number of repetitions have been reached, a first set is completed. A second set of repetitions may follow on the display, and the start of the set may be when an expected exercise is detected.

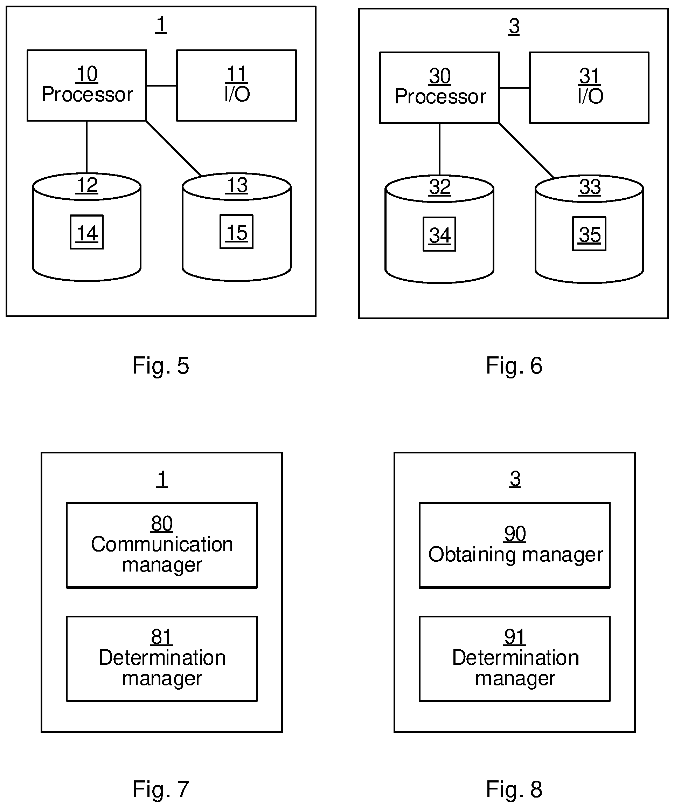

Fig. 5 is a schematic diagram showing some components of the handheld user device 1. The processing circuitry 10 may be provided using any combination of one or more of a suitable central processing unit, CPU, multiprocessing circuitry, microcontroller, digital signal processing circuitry, DSP, application specific integrated circuit, graphics processing unit, GPU, etc., capable of executing software instructions of a computer program 14 stored in a memory. The memory can thus be considered to be or form part of the computer program product 12. The processing circuitry 10 may be configured to execute methods described herein with reference to Fig. 2.

The memory may be any combination of read and write memory, RAM, and read only memory, ROM. The memory may also comprise persistent storage, which, for example, can be any single one or combination of magnetic memory, optical memory, solid state memory or even remotely mounted memory.

A second computer program product 13 in the form of a data memory may also be provided, e.g. for reading and/or storing data during execution of software instructions in the processing circuitry 10. The data memory can be any combination of read and write memory, RAM, and read only memory, ROM, and may also comprise persistent storage, which, for example, can be any single one or combination of magnetic memory, optical memory, solid state memory or even remotely mounted memory. The data memory may e.g. hold other software instructions 15, to improve functionality for the handheld user device 1.

The handheld user device 1 may further comprise an input/output (I/O) interface 11 including e.g. a user interface. The handheld user device 1 may further comprise a receiver configured to receive signalling from other devices, and a transmitter configured to transmit signalling to other devices (not illustrated). Other components of the handheld user device 1 are omitted in order not to obscure the concepts presented herein.

Fig. 6 is a schematic diagram showing some components of a cloud server 3. The processing circuitry or graphical processing unit 30 may be provided using any combination of one or more of a suitable central processing unit, CPU, multiprocessing circuitry, microcontroller, digital signal processing circuitry, DSP, application specific integrated circuit, graphics processing unit, GPU, etc., capable of executing software instructions of a computer program 34 stored in a memory. The memory can thus be considered to be or form part of the computer program product 32. The processing circuitry 30 may be configured to execute methods described herein with reference to Fig. 2.

The memory may be any combination of read and write memory, RAM, and read only memory, ROM. The memory may also comprise persistent storage, which, for example, can be any single one or combination of magnetic memory, optical memory, solid state memory or even remotely mounted memory.

A second computer program product 33 in the form of a data memory may also be provided, e.g. for reading and/or storing data during execution of software instructions in the processing circuitry 30. The data memory can be any combination of read and write memory, RAM, and read only memory, ROM, and may also comprise persistent storage, which, for example, can be any single one or combination of magnetic memory, optical memory, solid state memory or even remotely mounted memory. The data memory may e.g. hold other software instructions 35, to improve functionality for the handheld user device 3.

The cloud server 3 may further comprise an input/output (I/O) interface 31 including e.g. a user interface. The cloud server 3 may further comprise a receiver configured to receive signalling from other devices, and a transmitter configured to transmit signalling to other devices (not illustrated). Other components of the cloud server 3 are omitted in order not to obscure the concepts presented herein.

A database 33 of the cloud server 3 may be a position database used by the handheld user device 1.

According to an aspect, an embodiment of a handheld user device 1 for an advancement manager is presented with reference to Fig. 7. The handheld user device 1 comprises a communication manager unit 80 for providing a correction indication towards a UI of the handheld user device for verified incorrect performance. The handheld user device 1 also comprises a determination manager 81 for detecting an exercise of a person in front of a camera of the handheld user device, wherein the exercise is a trained exercise, for determining, when a stability of the detected exercise is within a first threshold, a performance of the detected exercise as correct, and otherwise determine the performance as incorrect, and for verifying that the performance determined as incorrect also occurs in subsequent repetition of the detected exercise before providing the correction indication.

Fig. 7 is a schematic diagram showing functional blocks of the handheld user device 1. The modules may be implemented as only software instructions such as a computer program executing in the cache server or only hardware, such as application specific integrated circuits, field programmable gate arrays, discrete logical components, transceivers, etc. or as a combination thereof. In an alternative embodiment, some of the functional blocks may be implemented by software and other by hardware. The modules correspond to the process blocks in the method illustrated in Fig. 2, comprising a communication manager unit 80 and a determination manger unit 81. In the embodiments where one or more of the modules are implemented by a computer program, it shall be understood that these modules do not necessarily correspond to process modules, but can be written as instructions according to a programming language in which they would be implemented, since some programming languages do not typically contain process modules.

The communication manager 80 is for an advancement manager in a handheld user device. This module corresponds to the processing blocks S120 and S150 of Fig. 2. This module can e.g. be implemented by the processing circuitry 10 of Fig. 5, when running the computer program.

The determination manager 81 is for an advancement manager in a handheld user device. This module corresponds to the processing blocks S100, S110, S130 and S140 of Fig. 2. This module can e.g. be implemented by the processing circuitry 10 of Fig. 5, when running the computer program.

Fig. 8 is a schematic diagram showing functional blocks of the cloud server 3. The modules may be implemented as only software instructions such as a computer program executing in the cache server or only hardware, such as application specific integrated circuits, field programmable gate arrays, discrete logical components, transceivers, etc. or as a combination thereof. In an alternative embodiment, some of the functional blocks may be implemented by software and other by hardware. The modules correspond to the process blocks in the method illustrated in Fig. 2, comprising an obtaining manager unit 90 and a determination manger unit 91. In the embodiments where one or more of the modules are implemented by a computer program, it shall be understood that these modules do not necessarily correspond to process modules, but can be written as instructions according to a programming language in which they would be implemented, since some programming languages do not typically contain process modules.

The obtaining manager 90 is for an advancement manager in a handheld user device. This module corresponds to the processing blocks S120 and S150 of Fig. 2. This module can e.g. be implemented by the processing circuitry 30 of Fig. 6, when running the computer program.

The determination manager 91 is for an advancement manager in a handheld user device. This module corresponds to the processing blocks S100, S110, S130 and S140 of Fig. 2. This module can e.g. be implemented by the processing circuitry 30 of Fig. 5, when running the computer program.

Computer programs have been describes as implemented in a handheld device or in a cloud server. One part of a computer programs may alternatively be implemented in the handheld device whereas other parts of the computer program may be implemented in the cloud server.

The aspects of the present disclosure have mainly been described above with reference to a few embodiments and examples thereof. However, as is readily appreciated by a person skilled in the art, other embodiments than the ones disclosed above are equally possible within the scope of the invention, as defined by the appended patent claims.

## Claims

1. A method for an advancement manager in a handheld user device, the method being performed in a handheld user device (1), and the method comprising:
- detecting (S100) an exercise of a person in front of a camera (46) of the handheld user device, wherein the exercise is a trained exercise;
- determining (S130), when a stability of the detected exercise is within a first threshold, a performance of the detected exercise as correct, and otherwise determine the performance as incorrect;
- verifying (S140) that the performance determined as incorrect occurs in a subsequent repetition of the detected exercise before providing a correction indication; and
- providing (S150) the correction indication towards a user interface, UI, of the handheld user device for the verified incorrect performance.

2. The method according to claim 1, further comprising:
- determining (S110) a weight of an exercise equipment present in the detected exercise, wherein the first threshold is dependent of the determined weight.

3. The method according to any one of claims 1 to 2, wherein the exercise is detected by image recognition.

4. The method according to any one of claims 1 to 3, wherein the stability is determined by detecting movement of one or more reference points of the person in front of the camera.

5. The method according to claim 4, wherein the first threshold is a distance unit in a first dimension based on a normalized value of a distance in a second dimension.

6. The method according to any one of claims 1 to 5, wherein the performance of the detected exercise is determined as correct when also a speed of the detected exercise is below a second threshold.

7. The method according to claim 6, wherein the second threshold is a predetermined value.

8. The method according to any one of claims 1 to 7, wherein a performance of the detected exercise is determined as correct when also a relative position of two or more reference points of the person in front of the camera is within a predetermined interval.

9. The method according to any one of claims 1 to 8, wherein the first threshold is dependent of an exercise history of the person in front of the camera.

10. The method according to any one of claims 1 to 9, wherein the correction indication is an image, or a video, displayed on a display of the handheld user device, and/or a voice message played by a speaker of the handheld user device.

11. The method according to any one of claims 1 to 10, further comprising:
- providing (S120) an advancement indication towards the UI for the detected exercise.

12. The method according to claim 11, wherein a set of repetitions of the detected exercise is started upon the detected exercise.

13. A handheld user device for an advancement manager, the handheld user device (1) comprising:
- a camera (46);
- a user interface, UI, (11);
- a processing circuitry (10);
- a computer program product storing instructions that, when executed by the processing circuitry, causes the handheld user device to:
- detect an exercise of a person in front of the camera, wherein the exercise is a trained exercise;
- determine, when a stability of the detected exercise is within a first threshold, a performance of the detected exercise as correct, and otherwise determine the performance as incorrect;
- verifying that the performance determined as incorrect occurs in a subsequent repetition of the detected exercise before providing the correction indication; and
- provide a correction indication towards the UI for the determined incorrect performance.

14. A computer program for an advancement manager in a handheld user device, the computer program comprising computer program code which, when run in a handheld user device (1), causes the handheld user device to:
- detect (S100) an exercise of a person in front of a camera (46) of the handheld user device, wherein the exercise is a trained exercise;
- determine (S130), when a stability of the detected exercise is within a first threshold, a performance of the detected exercise as correct, and otherwise determine the performance as incorrect;
- verify (S140) that the performance determined as incorrect occurs in a subsequent repetition of the detected exercise before providing the correction indication; and
- provide (S150) a correction indication towards a user interface, UI, of the handheld user device for the determined incorrect performance.
